# EUROPEAN PATENT APPLICATION

(11) **EP 3 848 062 A1**
(43) Date of publication of application: **14.07.2021**
(21) Application number: 21150692.8
(22) Date of filing: 08.01.2021
(51) Int. Cl.: A61L 15/22, A61L 15/46

(54) **A MEDICAL DEVICE FOR PREVENTING AND/OR TREATING INFECTIONS OF SKIN LESIONS**

(30) Priority: 10.01.2020 IT 202000000304
(71) Applicant: Fastmeditalia S.r.L., 20832 Desio (Monza Brianza) (IT); Zanolo S.p.A., 13852 Quaregna Cerreto (BI) (IT)
(72) Inventor: PINNA, Marco, I-21051 Arcisate (VA) (IT); FORMA, Ornella, I-21021 Voltorre di Gavirate (VA) (IT); TONANI, Alberto, I-13100 Vercelli (IT); NOVELLO, Andrea, I-13855 Valdengo (BI) (IT)
(74) Representative: Freyria Fava, Cristina

(57) **Abstract**

A medical device and an activating method thereof, wherein the medical device is suitable for preventing and/or treating an infection of a skin lesion and includes:
(i) a substrate consisting essentially of cellulose,
(ii) at least one cationic copolymer obtained by reaction of at least one polyamine, at least one polyamidoamine or a mixture thereof with at least one polyisocyanate, wherein the copolymer is anchored to the substrate, and optionally
(iii) at least one dermatologically acceptable amphoteric surfactant.

## Description

### Field of the invention

The present invention relates to a medical device intended to prevent and/or treat bacterial and/or fungal infections that occur at acute and/or chronic skin lesions.

### Technological background

In a healing skin lesion, fibroblasts take on the appearance of myofibroblasts. The appearance of myofibroblasts corresponds to the beginning of the compaction of the connective tissue and the contraction of the lesion.

In the event that the lesion is affected by a bacterial infection, the bacteria are floating, separate and easily eliminated through cleansing with physiological solution and applying a dressing suited to the bed of the lesion. When, on the other hand, the bacteria anchor themselves to the damaged tissue, becoming aggregated communities of slow-growing cells, they can begin to communicate with each other and generate a biofilm causing a critical colonization of the lesion itself. Biofilm is defined as a stable and complex aggregation of microorganisms that interact chemically with each other, promoting the secretion of an adhesive and protective polysaccharide matrix for the bacteria themselves.

The resistance to antibacterials, which characterizes the current global health panorama, is a big problem in treating skin lesions and, in particular, of chronic skin lesions which - as such - present a difficult healing process.

The general data show the presence of a biofilm in 60%-100% of skin lesions with difficulty in healing. While - on the one hand - the role played by the biofilm in the chronicity of skin lesions is still being studied, it is more and more likely that skin lesions that are difficult to heal present a biofilm inside and/or on their surface, the presence of which delays or prevents healing (see World Union of Wound Healing Societies - WUWHS -, Congress of Florence, Positioning document. Management of biofilm. Wounds International 2016).

Managing the biofilm in chronic skin lesions is, therefore, transformed into the main objective of their care.

### Summary of the invention

Taking these premises into consideration, the need is therefore felt for new medical devices capable of preventing and/or treating infections that occur at the level of skin lesions and, in particular, infections by microorganisms characterized by the ability to form a biofilm.

In accordance with the invention, the aforesaid object is achieved thanks to the solution specifically recalled in the attached claims, which form an integral part of the present description.

One embodiment of the present invention relates to a medical device suitable for preventing and/or treating an infection of a skin lesion including, preferably essentially consisting of:
(i) a substrate consisting essentially of cellulose and/or at least one polysaccharide,
(ii) at least one cationic copolymer obtained by reaction of at least one polyamine, at least one polyamidoamine or a mixture thereof with at least one polyisocyanate, wherein the copolymer is anchored to the substrate,
(iii) at least one dermatologically acceptable amphoteric surfactant, and optionally
(iv) zeolite.

In a further embodiment, the present invention concerns a method for activating a medical device for subsequent application to a skin lesion, the medical device being suitable for preventing and/or treating an infection of a skin lesion and including, preferably essentially consisting of:
(i) a substrate consisting essentially of cellulose and/or at least one polysaccharide,
(ii) at least one cationic copolymer obtained by reaction of at least one polyamine, at least one polyamidoamine or a mixture thereof with at least one polyisocyanate, the copolymer being anchored to the substrate, and optionally
(iii) zeolite,
wherein the method includes a step of impregnation of the medical device with at least one dermatologically acceptable amphoteric surfactant for subsequent application to a skin lesion.

### Detailed description of the invention

In the following description, there are numerous specific details to provide a thorough understanding of the embodiments. The embodiments may be implemented in practice without one or more of the specific details, or with other methods, components, materials, etc. In other cases, well-known structures, materials or operations are not shown or described in detail to avoid obscuring certain aspects of the embodiments.

Throughout the present specification, the reference to "an embodiment" or "embodiment" means that a particular configuration, structure, or characteristic described in connection with the embodiment is included in at least one embodiment. Therefore, the appearance of expressions "in a certain embodiment" or "in an embodiment" in various point throughout this specification does not necessarily always refer to the same embodiment. Moreover, the particular details, structures or characteristics can be combined in any suitable way in one or more embodiments.

The headings used here are used merely for convenience and do not interpret the object or meaning of the embodiments.

As anticipated at the beginning, the present invention aims to provide a medical device suitable for preventing and/or treating infections of a skin lesion, in particular in the condition in which microorganisms are capable of producing a biofilm. When the biofilm has formed, there is an increase in the colonies of microorganisms within a skin lesion, with programmed migration of the colonies, which conquer the surrounding and deeper tissues. Preventing and eliminating microorganisms and the biofilm from the lesion is therefore the priority in treating the lesion.

The present invention therefore relates to a medical device configured to resolve the aforesaid technical problem comprising, preferably essentially consisting of:
(i) a substrate essentially consisting of cellulose and/or at least one polysaccharide,
(ii) at least one cationic copolymer obtained by reaction of at least one polyamine, at least one polyamidoamine or a mixture thereof with at least one polyisocyanate, wherein the copolymer is anchored to the substrate,
(iii) at least one dermatologically acceptable amphoteric surfactant, and optionally
(iv) zeolite.

The medical device disclosed in the present application is able to remove bacteria and/or fungi present in the skin lesion by exerting a physical-mechanical attraction on bacteria and/or fungi detaching them from the skin lesion. The medical device does not exert any biocidal activity on such microorganisms, being free of any compound having a biocidal activity, like for example silver, cationic surfactant(s) or other antibiotic substance(s) that is(are) not chemically bound to the cationic copolymer.

In one embodiment, the substrate comes in the form of fibre, fabric or non-woven fabric.

In one embodiment, the substrate is essentially composed (i) of artificial cellulosic fibres (such as viscose, tencel, modal, lyocell, cellulose acetate), (ii) of natural cellulosic fibres (such as, for example, cellulose pulp, cotton, and the like), (iii) of polysaccharide fibres (such as alginate fibre, chitin fibre, chitosan fibre) or combinations thereof.

In one embodiment, the substrate is composed of 80% to 100% cellulose (natural, artificial or combinations thereof), in which the non-cellulosic portion is made up of polyesters, polyolefins, polyamides or their mixtures.

In one embodiment, the substrate consists of at least 80% to 100% of the at least one polysaccharide and wherein the remaining non-polysaccharide portion consists of polyesters, polyolefins, polyamides or mixtures thereof.

In one embodiment, the substrate has a weight between 40 and 200 g/m².

In one embodiment, the at least one polyamine is selected from amines, polyethylenepoly-(HEPA)penta ethylenesamine; amines, polyethylenepoly-(TETA)triethylene tetramine; amines, polyethylenepoly-(DETA)diethylene tetramine (see the ECHA - European Chemicals Agency website).

In one embodiment, the at least one polyamidoamine is selected from adipic acid/diethylenetriamine-epichlorohydrin resin copolymer, adipic acid/triethylenetriamine-epichlorohydrin resin copolymer, adipic acid/tetraethylenetriamine-epichlorohydrin resin copolymer.

In one embodiment, the polyisocyanate is selected from homopolymers or copolymers, dispersed in a water base, comprising at least one of the monomers hexamethylenediisocyanate (HDI), diphenylmethane diisocyanate (MDI), toluenediisocyanate (TDI), isophorone diisocyanate (IPDI). The at least one polyisocyanate contains the NCO reactive group in an amount comprised between 5% and 20% w/w. The polyisocyanates are - in fact - able to react with primary and secondary amino groups present in the structure of the at least one polyamine and/or of the at least one polyamidoamine and with the hydroxyl groups of the cellulose or of the polysaccharide(s). This cross-linking allows the molecular weight of the copolymer to be increased and its anchorage to the substrate. The resulting product has a slightly lipophilic character.

The copolymerization reaction between the at least one polyamine, the at least one polyamidoamine or their mixtures with the at least one polyisocyanate is carried out according to the normal copolymerization techniques belonging to the common general knowledge of the person skilled in the art. In particular, these techniques envisage the premixing of the polyisocyanate(s) with the polyamine or polyamidoamine polymer(s), the subsequent impregnation with this mixture of the substrate and the cross-linking that takes place when the temperature rises between 130 and 180°C.

In one embodiment, the cationic copolymer has monomeric units consisting of polyamine, polyamidoamine or mixtures thereof and of polyisocyanate.

The at least one amphoteric surfactant is a zwitterionic compound which acts as a cationic surfactant in an acid medium or as an anionic surfactant in an alkaline medium.

In one embodiment, the at least one amphoteric surfactant is selected from dodecyl betaine, tetradecyl betaine, hexadecyl betaine, cocoanphodiacetate, cocoiminodipropionate, dodecyliminodipropionate, cocamidopropyl betaine, cocamidopropyl dimethyl hydroxy sulfo betaine and lecithin or mixtures thereof. Preferably, the at least one amphoteric surfactant is selected from dodecyl betaine, tetradecyl betaine, hexadecyl betaine, cocamidopropyl betaine, and cocamidopropyl dimethyl hydroxy sulfobetaine or mixtures thereof.

Application of other surfactant(s) with a charge different from the charge of amphoteric surfactant(s) did not give a positive result in terms of efficacy. As it is reported in the experimental part, the use of non-ionic or anionic surfactant(s) provide(s) a lower efficacy in the absorption of the bacterial membrane on the medical device, probably due to partial neutralization of the positive charge present on the cationic copolymer. The use of cationic surfactant(s) was not considered because they substances are toxic to bacteria, i.e. they act as a biocide and can be released on the skin lesion.

In one embodiment, in case the medical device comprises also zeolite, the zeolite is present in an amount comprised between 0.1 and 10% by weight with respect to the substrate. The zeolite is - in fact - able to retain the at least one amphoteric surfactant, allowing the device to be preserved for long periods of time and releasing the surfactant at the time of application on the skin lesion, thus avoiding the impregnation operation of the device with the at least one surfactant before its application.

The device is not cytotoxic and is compatible with mucous membranes and the injured human dermis.

The medical device subject of the present description is able to remove (by physical mechanical uptake) bacteria and/or fungi present in skin lesions or in perilesional tissue, where the lesions are, for example, wounds (surgical or due to trauma), ulcers, dehiscences, fasciotomies or erosions.

The medical device subject of the present description is able to exert its action of removing the microorganisms (be they bacteria and/or fungi) present on the surface of the lesion, attracting them electrostatically and blocking them to the substrate without exerting any biocidal action on them.

The substrate (consisting essentially of cellulose and/or at least one polysaccharide comprising a cationic copolymer on its surface obtained by cross-linking reaction between the at least one polyamine, the at least one polyamidoamine or their mixtures with at least one polyisocyanate) presents - by virtue of the aforesaid copolymer - a surface that is at least partially lipophilic. This lipophilia, which increases as the molecular weight of the copolymer increases, allows an interaction with the cell wall of the bacteria, which is also essentially lipophilic. Without wishing to be limited to any theory in this regard, but taking into consideration the numerous publications that generally support the ability of a cationic polymer to attract the bacterial membrane, the present inventors have reason to believe that the bacteria and biofilm disrupted by the use of an aqueous solution based on an amphoteric surfactant, allows the latter to act as a carrier in an aqueous medium, suspending the bacteria and the biofilm by means of its mild cationic charge and transporting them towards the absorbent substrate. The presence of a high positive charge present on the substrate thanks to the presence of the cationic copolymer anchored thereto is able to create Van der Waals bonds (i.e. weak electrostatic interactions). The bacteria are thus adsorbed and trapped on the surface of the substrate thanks to the dynamic contact (determined by rubbing) between the lesion and the substrate of the medical device.

Without the aid of an amphoteric surfactant (carrier), the substrate comprising the cationic copolymer would not be able to interact effectively with the bacterial membrane.

The combination of a cationic copolymer anchored to the solid substrate and an amphoteric surfactant allows very high bacterial removal values to be obtained, ensuring an excellent level of safety for the medical use of the device.

The present invention considers the presence of an amphoteric surfactant to be a necessary parameter for correct functioning of the medical device. The amphoteric surfactant is - in fact - able to break up the biofilm by suspending the bacteria and cellular debris, while cleaning the lesion. Thanks to the alkyl chain, the amphoteric surfactant is in fact able to penetrate inside the biofilm, to suspend and transport with its mild cationic charge both the bacteria and the biofilm onto the substrate provided with positive charges (thanks to the presence of the cationic copolymer). The bacteria and/or biofilm are thus adsorbed and trapped in the substrate and removed from the lesion.

The amphoteric surfactant can be directly applied to the medical device at the time of its production or it can be applied to the device at the time of use, before being applied to the lesion to be treated.

According to an embodiment, the present invention therefore refers to a method for activating a medical device for subsequent application to a skin lesion, the medical device being suitable for preventing and/or treating an infection of a skin lesion and including, preferably essentially consisting of:
(i) a substrate consisting essentially of cellulose and/or at least one polysaccharide,
(ii) at least one cationic copolymer obtained by reaction of at least one polyamine, at least one polyamidoamine or a mixture thereof with at least one polyisocyanate, the copolymer being anchored to the substrate, and optionally
(iii) zeolite,
wherein the method includes a step of impregnation of the medical device with at least one dermatologically acceptable amphoteric surfactant for subsequent application to a skin lesion. The at least one amphoteric surfactant is selected from dodecyl betaine, tetradecyl betaine, hexadecyl betaine, cocoanphodiacetate, cocoiminodipropionate, dodecyliminodipropionate, cocamidopropylbetaine, cocamidopropyl dimethyl hydroxy sulfo betaine and lecithin. Preferably, the at least one amphoteric surfactant is selected from dodecyl betaine, tetradecyl betaine, hexadecyl betaine, cocamidopropyl betaine, and cocamidopropyl dimethyl hydroxy sulfobetaine, or mixtures thereof.

This medical device, as the experimental data prove, is not included in the list of biocides, but is an article capable of exerting a physical-mechanical action on bacteria. In particular, the positive charges possessed by the at least one cationic copolymer applied on the substrate are exploited which - in the presence of an amphoteric surfactant - is capable of generating an attraction effect on the cell wall of the bacteria. As already mentioned, this attraction occurs through differences in potential charges between the aforesaid bacterial cell wall and/or the biofilm (weak electrostatic attractions, i.e. Van der Waals forces) and the surface of the medical device. Since the cationic copolymer is anchored to the solid substrate, no reaction substance or by-product interacts with the microorganism, nor is it released on the lesion. The substrate of the present invention therefore physically retains the microorganisms on its surface, maintaining their vitality unaltered.

### Preparation of a medical device according to the present invention

A sample of non-woven (NW) fabric made of 100% viscose (weighing 120 g/m²) was impregnated using padding with a water-based solution comprising 100 g/l of poly(dimethylamine-co-epichlorohydrin-co-ethylenediamine) and 5 g/l of a polyfunctional isocyanate on a base of blocked toluene diisocyanate (Imprafix® TH solution). The two polymers were premixed together with water and the impregnated NW fabric was subsequently heated to 170°C for 60 seconds. The final amount of linked polymer on the NW fabric varies from 1 to 3% w/w of NW fabric.

The medical device thus obtained is then cut into the formats suitable for the application needs of the product itself.

### In vitro efficacy analysis

To demonstrate the bacterial reduction in a solution in dynamic contact with the medical device made as described above, an aqueous solution was prepared with the bacterial strain *Staphylococcus aureus* (gram-positive bacterium; ATCC 6538).

In Table 1, 50 grams of bacterial suspension at a concentration of 10⁵ cells was subjected to dynamic contact with 1 gram of the medical device in the presence of cocamido-propyl-betaine (an amphoteric surfactant).

**Table 1**

| **Sample name** | **Temperature** | **Dynamic contact time** | **Bacterial reduction %** |
|---|---|---|---|
| Medical device + 2 g/l of cocamidopropyl betaine | environment | 1 hour | 100 |
| 2 g/l of cocamidopropyl betaine | environment | 1 hour | 12 |

The data reported in Table 1 show a total reduction of the bacterial load in the liquid in contact with the medical device subject of the present invention. Conversely, there is an insignificant reduction in the bacterial load (equal to only 12%) in the control liquid in the presence of cocamido-propyl-betaine alone.

Similar results have also been obtained with reference to bacteria *Escherichia coli* (gram-negative bacteria; ATCC 11229).

The same experiment was then carried out using no surfactant at all (Table 2) or non-ionic or anionic surfactants (Tables 3 and 4).

**Table 2**

| **Sample name** | **Temperature** | **Dynamic contact time** | **Bacterial reduction %** |
|---|---|---|---|
| Medical device without amphoteric surfactant | environment | 1 hour | 65 |

The data reported in Table 2 show a partial reduction of *Staphylococcus aureus* in the liquid in contact with the medical device subject of the present invention. Without wishing to be bound to any specific theory, the present inventors have reason to believe that the cationic copolymer, due to its partially lipophilic character, is not able to come into contact with the bacteria and it is therefore unable to absorb them on the surface of the medical device.

**Table 3**

| **Sample name** | **Temperature** | **Dynamic contact time** | **Bacterial reduction %** |
|---|---|---|---|
| Medical device + 2 g/l of nonionic surfactant TWEEN20 (Polyoxyethylenesorbitan monolaurate) | environment | 1 hour | 68 |
| 2 g/l of nonionic surfactant TWEEN20 (Polyoxyethylenesorbitan monolaurate) | environment | 1 hour | 5 |

**Table 4**

| **Sample name** | **Temperature** | **Dynamic contact time** | **Bacterial reduction %** |
|---|---|---|---|
| Medical device + 2 g/l of anionic surfactant Sodium dodecilsulphate (SDC) | environment | 1 hour | 52 |
| 2 g/l of anionic surfactant Sodium dodecilsulphate (SDC) | environment | 1 hour | 16 |

The data reported in Tables 3 and 4 show a partial reduction of *Staphylococcus aureus* in the liquid in contact with the medical device subject of the present invention; such a reduction is in any case much lower (68 and 52%) than the reduction obtained using an amphoteric surfactant (100% as witnessed in Table 1). The reduction in bacterial load in the control liquid observed using the non-ionic surfactant (Tween20) or the anionic surfactant (SDS) alone (i.e. without the medical device) is absolutely insignificant being only 5% and 16%, respectively.

The data provided in the above tables clearly demonstrate that the use of an amphoteric surfactant is important to the effectiveness of the medical device subject of the present invention, as it likely acts as a "bacterial carrier" that drives bacteria from the liquid to the medical device.

### Verification of the non-migration of biocidal substances from the medical device

To verify the ability of the medical device not to release substances capable of inhibiting bacterial load, the ISO 20645/2004 test was performed according to the international standard method (Agar diffusion plate test) . This test verifies whether a white and transparent halo is generated on the plate used for the test around the point where the biocidal agent is seeded; in this condition, the microorganism responsible for the infection is sensitive to the biocidal agent. If, however, the colonies of the micro-organism continue to extend, this means that it is resistant to the biocidal agent used.

The medical device of the present invention and a control sample represented by a standard cotton fabric were used in this bacterial reduction test against *Staphylococcus aureus.*

The results show the lack of inhibition halo around the medical device subject of the present invention and around the control sample.

It is therefore demonstrated that the copolymer present on the medical device of the present invention does not release biocidal substances capable of spreading through the agar.

### Analysis of the efficacy of the medical device in vivo

The medical device subject of the present invention can be applied on any type of lesion (acute or chronic) of different aetiology: contaminated, colonized, in critical colonization, full-blown infection.

Pathogenic bacteria and fungi present on the lesion are captured by the medical device through physical action. The medical device is activated by applying thereon a cleaning solution comprising at least one amphoteric surfactant, which allows direct action on the bacteria/fungi by preventing and/or reducing biofilm formation.

The bacteria have hydrophobic characteristics and are captured by the medical device, in this way a bacteriostatic action is caused by acting on the number of bacteria by changing the medical device. The more the wound is exuding, the more the changes of the medical device must be intensified (from 24 to 72 hours).

The medical device does not release any type of substance on the wound bed (antibiotics and/or antiseptics), avoiding any risk of developing resistant bacterial strains, thus not interfering with the wound healing process. This medical device, therefore, does not destroy bacteria, avoiding the release of endotoxins (direct action of silver-releasing dressings) which may cause ineffective inflammation.

Instructions for treating a wound with the medical device:
1. after the hygiene, clean the bottom of the lesion and the perilesional skin with cleaning solution (possibly containing an amphoteric surfactant) leaving a humidity gradient with the same solution;
2. perform a compress with cleaning or antiseptic solution optionally containing at least one amphoteric surfactant;
3. impregnate the medical device with at least one amphoteric surfactant;
4. place the impregnated medical device in direct contact with the lesion bed and perilesional skin for effective collection and absorption of bacteria, which become imprisoned in the medical device itself. In the presence of cavitary lesions, use the medical device as a swab.

Depending on the amount of exudate, secondary dressings with absorbent action can be used.

The medical device subject of the present invention can also be used as a filler associated with "Negative Topical Pressure".

The medical device according to the present invention has been clinically tested on 11 patients with the following diagnoses:
- laparotomy wound dehiscence;
- internal malleolar ulcer;
- left heel polytrauma;
- sternum dehiscence;
- dehiscence with lower right limb fasciotomy;
- sternal dehiscence;
- left leg venous ulcer;
- left leg venous ulcer;
- ischemic ulcer;
- lower limb venous ulcers;
- left leg lateral fasciotomy.

Following application of the medical device the following was observed:
- a drastic reduction in inflammation of the perilesional skin with significant reduction from the first device change at 72 hours (for all 11 patients);
- a reduction of the microbial load on the bottom of the lesion, on the edges and on the margins (documented with the Moleculight device) of the MolecuLight Corp. supplier. 2403 Sidney Street, Suite 209 Pittsburgh.
- a collection of bacteria inside the medical device (documented with the Moleculight device); and
- a reduction in pain and odour felt by the patient.

## Claims

1. A medical device suitable for preventing and/or treating an infection of a skin lesion including:
(i) a substrate consisting essentially of cellulose and/or at least one polysaccharide,
(ii) at least one cationic copolymer obtained by reaction of at least one polyamine, at least one polyamidoamine or a mixture thereof with at least one polyisocyanate, wherein the copolymer is anchored to the substrate,
(iii) at least one dermatologically acceptable amphoteric surfactant, and optionally
(iv) zeolite.

2. The medical device according to claim 1, wherein the substrate is in the form of fibre, fabric or non-woven fabric.

3. The medical device according to claim 1 or claim 2, wherein the cellulose is selected from artificial cellulose, natural cellulose and combinations thereof.

4. The medical device according to any one of the preceding claims, wherein the substrate is composed of 80% to 100% cellulose and wherein the remaining non-cellulosic portion consists of polyesters, polyolefins, polyamides or mixtures thereof.

5. The medical device according to any one of the preceding claims, wherein the at least one polysaccharide is selected from alginate, chitin and chitosan.

6. The medical device according to any one of the preceding claims, wherein the substrate consists of at least 80% to 100% of the at least one polysaccharide and wherein the remaining non-polysaccharide portion consists of polyesters, polyolefins, polyamides or mixtures thereof.

7. The medical device according to any one of the preceding claims, wherein the at least one polyamine is selected from amines, polyethylenepoly-pentaethylenesamine; amines, polyethylenepoly-triethylentetramine; amines, polyethylenepoly-diethylentetramine.

8. The medical device according to any one of the preceding claims, wherein the at least one polyamidoamine is selected from adipic acid/diethylenetriamine- epichlorohydrin resin copolymer, adipic acid/triethylenetriamine-epichlorohydrin resin copolymer, adipic acid/tetraethylenetriamine-epichlorohydrin resin copolymer.

9. The medical device according to any one of the preceding claims, wherein the at least one polyisocyanate contains at least 5% to 20% w/w of NCO groups.

10. The medical device according to any one of the preceding claims, wherein the at least one polyisocyanate is selected from homopolymers or copolymers containing at least one among the monomers hexamethylenediisocyanate (HDI), diphenylmethane diisocyanate (MDI), toluenediisocyanate (TDI), and isophorone diisocyanate.

11. The medical device according to any one of the preceding claims, wherein the at least one amphoteric surfactant is selected from dodecyl betaine, tetradecyl betaine, hexadecyl betaine, cocoanphodiacetate, cocoiminodipropionate, dodecyliminodipropionate, cocoamidopropylbetaine, cocoamidopropyl dimethyl hydroxy sulfo betaine, lecithin and mixtures thereof.

12. The medical device according to any one of the preceding claims, wherein the zeolite is present in a quantity between 0.1 and 10% w/w of the substrate.

13. A method for activating a medical device for subsequent application to a skin lesion, the medical device being suitable for preventing and/or treating an infection of a skin lesion and including:
(i) a substrate consisting essentially of cellulose and/or at least one polysaccharide,
(ii) at least one cationic copolymer obtained by reaction of at least one polyamine, at least one polyamidoamine or a mixture thereof with at least one polyisocyanate, the copolymer being anchored to the substrate, and optionally
(iii) zeolite,
wherein the method includes a step of impregnation of the medical device with at least one dermatologically acceptable amphoteric surfactant.

14. The method according to claim 13, wherein the at least one amphoteric surfactant is selected from dodecyl betaine, tetradecyl betaine, hexadecyl betaine, cocoanphodiacetate, cocoiminodipropionate, dodecyliminodipropionate, cocamidopropylbetaine, cocamidopropyl dimethyl hydroxy sulfo betaine, lecithin and mixtures thereof.
